(19) Europäisches Patentamt / European Patent Office / Office européen des brevets

(11) **EP 4 235 146 B1**

(12) **EUROPEAN PATENT SPECIFICATION**

(45) Date of publication and mention of the grant of the patent:
**02.09.2026 Bulletin 2026/36**

(21) Application number: **23161319.1**

(22) Date of filing: **13.03.2018**

(51) International Patent Classification (IPC):
***G01N 21/64*** *(2006.01)* ***B01L 3/00*** *(2006.01)*
***G01N 37/00*** *(2006.01)* ***G01N 15/06*** *(2024.01)*
*G01N 21/03* *(2006.01)* *G01N 15/01* *(2024.01)*
*G01N 15/075* *(2024.01)*

(52) Cooperative Patent Classification (CPC):
**G01N 21/6452; B01L 3/502715; G01N 15/06; G01N 37/00;** B01L 2200/0642; B01L 2200/0689; B01L 2300/0809; B01L 2300/0829; B01L 2300/0877; B01L 2300/0887; B01L 2300/1805; G01N 15/01; G01N 15/075; G01N 2015/0687; G01N 2021/0325

(54) **ANALYSIS DEVICE, ANALYSIS KIT, AND ANALYSIS SYSTEM**

ANALYSEGERÄT, ANALYSEKIT UND ANALYSESYSTEM

DISPOSITIF D’ANALYSE, KIT D’ANALYSE ET SYSTÈME D’ANALYSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.03.2017 JP 2017048601**

(43) Date of publication of application:
**30.08.2023 Bulletin 2023/35**

(62) Document number(s) of the earlier application(s) in accordance with Art. 76 EPC:
**18766867.8 / 3 598 104**

(73) Proprietor: **Toppan Printing Co., Ltd.**
**Tokyo 110-0016 (JP)**

(72) Inventors:
- **NAGAHARA, Misaki**
  **TOKYO 110-0016 (JP)**
- **MAKINO, Yoichi**
  **TOKYO 110-0016 (JP)**
- **HOSHINO, Akihiro**
  **TOKYO 110-0016 (JP)**
- **HIRASE, Takumi**
  **TOKYO 110-0016 (JP)**
- **KUNITOMI, Tomoko**
  **TOKYO 110-0016 (JP)**
- **GOTO, Keisuke**
  **TOKYO 110-0016 (JP)**
- **HORIUCHI, Yosuke**
  **TOKYO 110-0016 (JP)**

(74) Representative: **Plasseraud IP**
**104 Rue de Richelieu**
**CS92104**
**75080 Paris Cedex 02 (FR)**

(56) References cited:
**WO-A1-00/71991** **WO-A1-2016/196229**
**JP-A- 2017 049 151** **US-A1- 2003 044 967**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

[Technical Field]

The present invention relates to an analysis device, an analysis kit, and an analysis system.

[Background Art]

Recently, diagnostic approaches based on detection of biomarkers are gaining attention. In these approaches, biomarkers (e.g., nucleic acids such as DNA and RNA, or proteins) contained in a patient's body fluids, such as blood, are detected to diagnose cancers or Alzheimer's disease, or other diseases, such as gene disorders, or infectious disorders due to bacteria or the like.

As methods of measuring biomarkers, there are known biomarker assays based on a digital counting method, such as digital ELISA, digital PCR assay, and digital Invader assay. The digital counting method refers to a method of detecting a concentration of biomarkers by distributing biomarkers into a number of fine wells (e.g., each having a dimension of several micrometers) on a one-to-one basis and counting the wells holding the biomarkers.

The digital counting method may be a method in which target substances (objects to be detected), i.e. biomarkers, are respectively bound to particles and a number of such particles are encapsulated in respective wells to detect the number of target substances (the number of wells holding the target substances).

For example, PTL 1 describes that target substances are respectively bound to magnetic beads (particles), and a sample fluid containing a number of such magnetic beads is supplied into a channel in which a number of fine wells are open so that the magnetic beads are respectively distributed to the number of wells. PTL 1 also describes that, after distributing the magnetic beads to the wells, a reagent fluid (detection reagent), such as a fluorogenic substrate (RGP), is supplied to the channel. The reagent in the fluid is an enzyme fluid or the like that reacts with target substances to create a fluorescence phenomenon, and is used for counting wells holding the target substances. PTL 2 discloses a method of accurately detecting a signal from a minute well put with a bead when using an array device for biological component nucleic acid. PTL 3 discloses a method for RNA quantification of known type.

[Citation List]

[Patent Literature]

PTL 1: JP 2014-503831 T
PTL 2: JP 2017 049151 A1
PTL 3: Wo 2016/196229 A1

[Summary of the Invention]

[Technical Problem]

The digital counting method using liquid biopsy uses a patient's body fluids, such as blood. In this method, target substances are less likely to be detected as the concentration of the target substances becomes lower. Therefore, a method with which such low-concentration target substances can be detected has been sought.

The present invention has been made in view of the above circumstances, and aims to provide an analysis device, an analysis kit, and an analysis system with which low-concentration target substances can be detected.

[Solution to Problem]

According to the invention, there is provided an analysis device for detecting biochemical reactions using a fluorescence device as defined with appended independent claim 1. The device comprises, among other features as defined with claim 1, a well array (4) in which a plurality of wells (3) are formed; and each of the wells has a volume of 100 fL to 5,000 fL,

each of the wells (3) has a surface area ratio (surface area of well / volume of well) of 1 or less; and
each of the wells (3) has an aspect ratio of 1 or more as calculated by the following Formula (1)

Aspect ratio = Depth of each well / Diameter of largest circle among circles included in a region enclosed by a rim of each well.

Preferred embodiments are defined with the dependent claims. Each of the wells may have a depth that is larger than a depth of field of the fluorescence microscope and 100 times or less the depth of field.

The depth of each of the wells may be 3 times or more the depth of field and 10 times or less the depth of field.

An analysis kit may be defined which comprises the analysis device, a detector, a detection reagent (23), and a sealing liquid (24).

There is also described an analysis system comprising the above analysis kit, the fluorescence microscope, and an analysis unit (41) that analyzes values measured by the fluorescence microscope.

The present invention may be construed to include the following aspects.

In an aspect as defined with appended independent claim 1, the present invention is an analysis device for detecting a biochemical reaction by using a detector. The device includes (among other features as defined with claim 1) a well array (microwell array) in which a plurality of wells are formed. Each of the wells has a volume that is 100 fL or more, and a depth that is larger than a depth of field of the device and 100 times or less the depth of field.

In the analysis device, the depth of each of the wells may be 3 times or more the depth of field and 10 times or less the depth of field.

In the analysis device, each of the wells may have a depth/aperture diameter ratio of 1 or more.

In the analysis device, the biochemical reaction may be a heating reaction.

In an aspect, the present invention is an analysis kit including the analysis device set forth above, a detection reagent, and a sealing liquid.

In an aspect, the present invention is an analysis system including the analysis kit set forth above, the detector, and an analysis unit that analyzes values measured by the detector.

[Advantageous Effects of the Invention]

The analysis device, the analysis kit and the analysis system set forth above can detect low-concentration target substances.

[Brief Description of the Drawings]

Fig. 1 is a perspective view illustrating a microfluidic device according to an embodiment of the present invention.
Fig. 2 is a cross sectional view taken along the line II-II of Fig. 1.
Fig. 3 is a set of diagrams illustrating a principal part of a microwell array of the microfluidic device.
Fig. 4 is a diagram illustrating an example of use of the microfluidic device.
Fig. 5 is a diagram illustrating an example of use of the microfluidic device.
Fig. 6 is a diagram illustrating an example of use of the microfluidic device.
Fig. 7 is a set of schematic diagrams each illustrating a well of a microwell array and a depth of field of a detector.
Fig. 8 is an image of a fluorescence field related to Example 1.
Fig. 9 is an image of a fluorescence field related to Comparative Example 1.
Fig. 10 is a scanning electron micrograph of a well array of an analysis device according to Example 3.
Fig. 11 is a scanning electron micrograph of a well array of an analysis device according to Example 4.
Fig. 12 is a scanning electron micrograph of a well array of an analysis device according to Example 5.
Fig. 13 is a scanning electron micrograph of a well array of an analysis device according to Comparative Example 3.
Fig. 14 is a scanning electron micrograph of a well array of an analysis device according to Comparative Example 4.
Fig. 15 is a set of images in which (a) shows typical fluorescence signals detected by using an analysis device of Example 3, (b) shows typical fluorescence signals detected by using an analysis device of Example 4, (c) shows typical fluorescence signals detected by using an analysis device of Example 5, (d) shows typical fluorescence signals detected by using an analysis device of Comparative Example 3, and (e) shows typical fluorescence signals detected by using an analysis device of Comparative Example 4.

[Description of Embodiments]

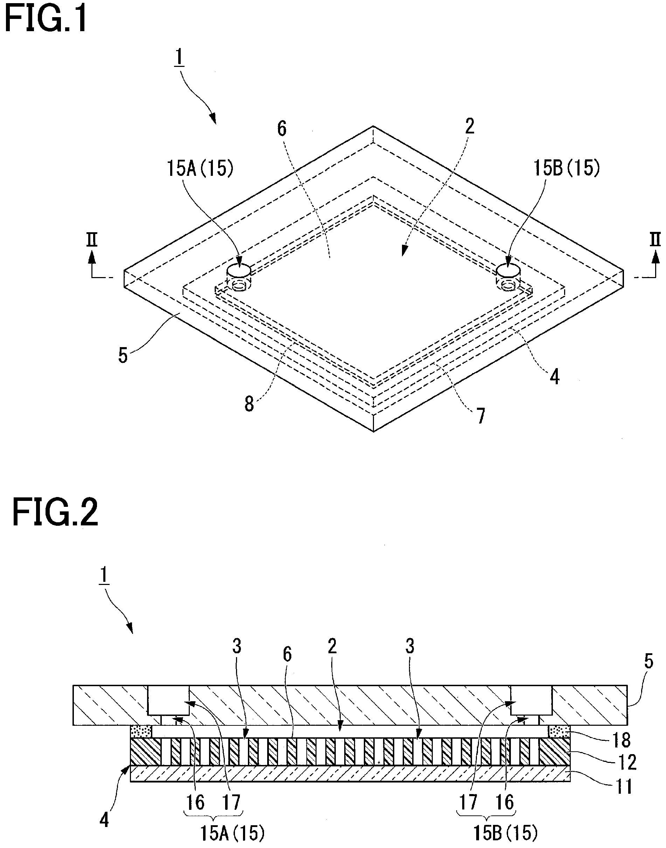

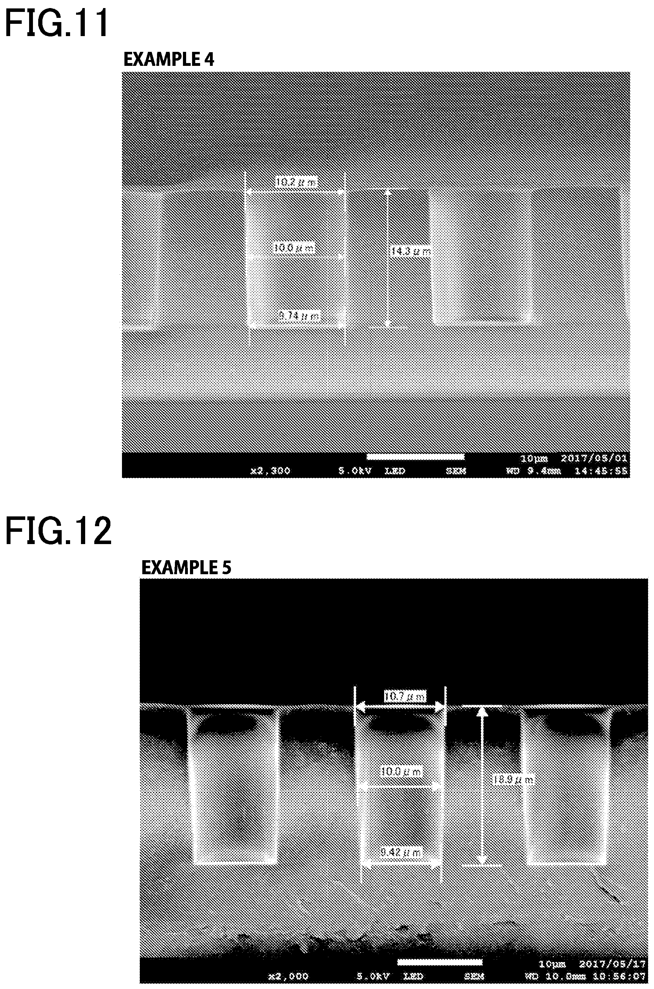

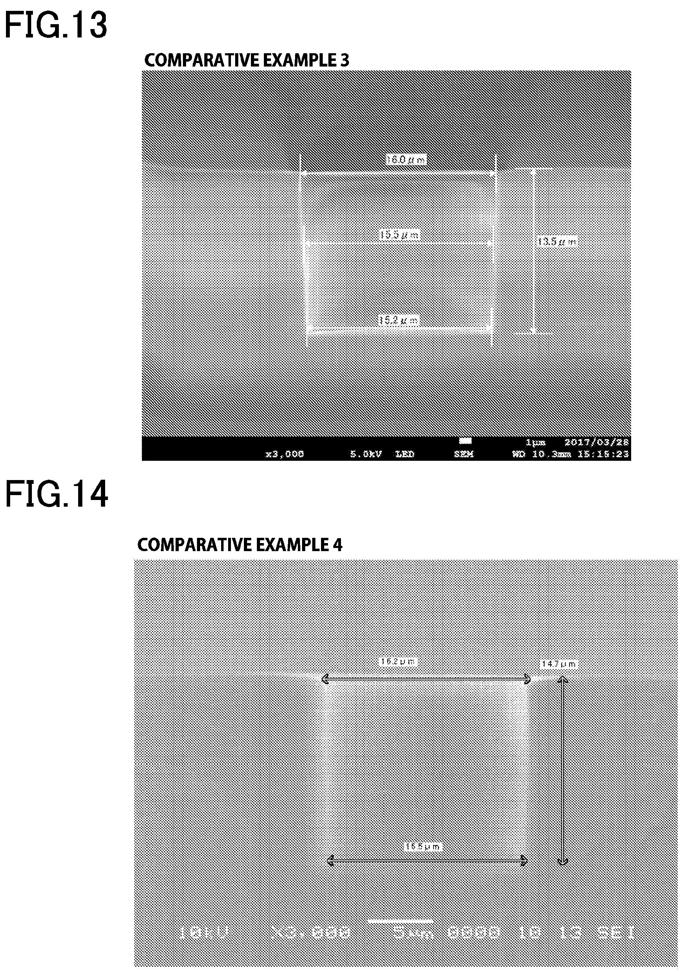

With reference to Figs. 1 to 15, an analysis device, an analysis kit and an analysis system according to an embodiment of the present invention will be described.

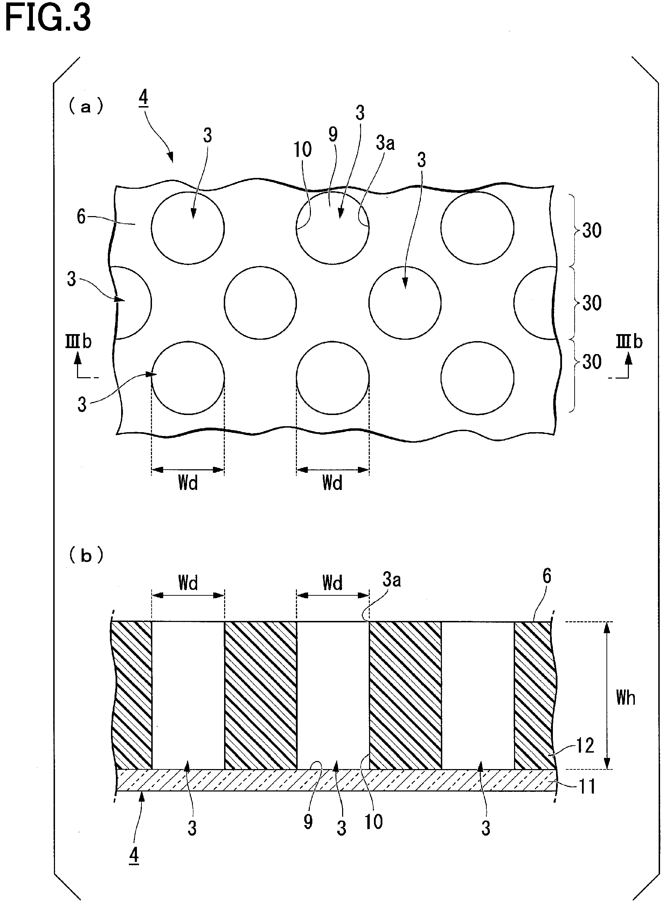

Firstly, an analysis device of the present embodiment will be described. This analysis device is a microfluidic device 1 which is used for a digital counting method that uses liquid biopsy. Fig. 1 is a perspective view illustrating the

microfluidic device 1. Fig. 2 is a cross sectional view taken along the line II-II of Fig. 1. Fig. 3 is a set of diagrams illustrating a principal part of a microwell array 4 of the microfluidic device 1. Fig. 3(a) is a plan view of the microfluidic device 1, and Fig. 3(b) is a cross-sectional view taken along the line IIIb-IIIb of Fig. 3(a). It should be noted that the microfluidic device 1 can be used not only for liquid biopsy, but also for measurement of proteins, DNA, RNA, viral particles, bacteria, or the like.

As shown in Figs. 1 to 3, the microfluidic device 1 is provided with a microwell array 4 in which a plurality of wells 3 are formed. As shown in Fig. 3(a), the plurality of wells 3 of the microwell array 4 are arranged in an array pattern. In Fig. 3(a), the plurality of wells 3 are arrayed in the lateral direction (in the horizontal direction as viewed in Fig. 3(a)) with an interval therebetween. A line of wells 3 arrayed in the lateral direction form a lateral-array well group 30. A plurality of such lateral-array well groups 30 are arranged in the longitudinal direction (in the vertical direction as viewed in Fig. 3(a)). In lateral-array well groups 30 adjacently located in the longitudinal direction, wells 3 are arranged being offset in the lateral direction so as not to overlap with each other. The plurality of wells 3 of the microwell array 4 preferably all have the same shape.

The microwell array 4 has a flat surface in which the plurality of wells 3 are open (aperture surface 6). The plurality of wells 3 may be formed on the overall aperture surface 6 of the microwell array 4. In the present embodiment, however, the plurality of wells 3 are formed in a region (well-forming region 8), excepting a perimeter region 7, of the aperture surface 6 of the microwell array 4. The well-forming region 8 may have any contour. In the present embodiment, the contour is rectangular.

As shown in Figs. 2 and 3, each well 3 is a hole having a bottom. In the present embodiment, each well 3 is cylindrically formed and has a bottom 9. It should be noted that the well 3 may have any shape other than the cylindrical shape. For example, besides the cylindrical shape, the well 3 may have a polygonal shape defined by a plurality of faces (e.g., rectangular parallelepiped, or six- or eight-sided prism), an inverse conical shape, an inverse pyramidal shape (inverse three-, four- five- or six-sided pyramidal shape, or inverse seven or more-sided polygonal pyramidal shape), or the like. The inverse conical or pyramidal shape refers to a shape of the well 3 in which the bottom of the cone or the pyramid serves as an aperture of the well 3. If the well 3 has a reverse conical or pyramidal shape, the vertex, for example, of the cone or the pyramid may be cut off to make the bottom 9 of the well 3 flat. As another example, the well 3 may have a bottom 9 which is recessed or protruded relative to the aperture of the well 3. Also, the well 3 may have a shape, for example, that is a combination of two or more shapes mentioned above. For example, the well 3 may have a shape that is partially cylindrical, with the rest being reverse conical.

Accordingly, the well 3 may have a constant cross-sectional area or different cross-sectional areas in planes perpendicular to the depth direction of the well. Also, the well 3 may have a circular cross-sectional shape or any other cross-sectional shape in a plane perpendicular to the depth direction of the well. Moreover, in planes perpendicular to the depth direction of the well, the well 3 may have a constant cross-sectional shape throughout the depth of the well, or may have different cross-sectional shapes depending on the depth.

As shown in Fig. 3(b), each well 3 of the present embodiment has a depth (well depth) Wh that is larger than an aperture diameter Wd of the well 3. Specifically, a ratio of well depth Wh / aperture diameter Wd is greater than 1.

In other words, each well 3 has an aspect ratio of 1 or more as calculated from the following Formula (1).

Aspect ratio = Well depth / Diameter of the largest circle among the circles in a region enclosed by the brim of the well in plan view ... (1) (1)

As mentioned above, in planes perpendicular to the depth direction of the well, the wells 3 may each have a constant cross-sectional shape throughout the depth of the well, or may have different cross-sectional shapes depending on the depth. In the present specification, in either of the cases, the diameter used for calculating an aspect ratio is the diameter of the largest circle among the circles in a region enclosed by the rim of the well in plan view of the aperture of the well. The term rim herein refers to an edge of the aperture of each well.

For example, if each well has a cylindrical shape, the "diameter of the largest circle among the circles in a region enclosed by the rim of the well in plan view" can be the diameter of the aperture of the well.

As will be described later in examples, if each well configuring the well array of the analysis device has an aspect ratio of 1 or more, low-concentration target substances can be favorably detected. The expression "favorably detected" refers to that, for example, the signal/noise ratio is high. The term signal refers to a signal generated by an intended biochemical reaction, while the term noise refers to a signal generated by a negative control biochemical reaction that does not generate a signal. For example, the noise may be a signal generated by reaction of a detection reagent containing no objects to be detected, and may be termed background in other words.

The wells configuring the well array of the analysis device may each have an aspect ratio, for example, of 1 or more, or 1.3 or more, or 1.7 or more, or 2 or more, or 3 or more, or 3.5 or more, or 4 or more, as calculated from Formula (1) set forth above. However, higher aspect ratio may make the production of the well array more difficult. Therefore, the aspect ratio may have an upper limit which is, for example, 10 or 5 or 4.

In the following description, the "diameter of the largest circle among the circles in a region enclosed by the rim of the well in plan view" expressed in Formula (1) may be referred to as an "aperture diameter of the well".

Each well 3 has an aperture diameter Wd, for example, of 100 μm or less. The well depth Wh refers to a largest distance between a rim 3a and the bottom 9 of the well 3, in a direction perpendicular to the aperture surface 6. The aperture diameter Wd refers to the diameter of a largest circle among the circles in a region enclosed by the rim 3a in plan view. Since the well 3 has a cylindrical shape in the present embodiment, the well depth Wh corresponds to the length of the cylindrical shape in the axial direction, and the aperture diameter Wd corresponds to the diameter of the cylindrical shape. For example, if the well 3 has an inverse three-sided pyramidal shape, the well depth Wh corresponds to the height of the inverse three-sided pyramid, i.e. a distance between the triangular bottom and the vertex that is not included in the bottom, in the direction perpendicular to the bottom, and the aperture diameter Wd corresponds to the diameter of the circle inscribing the triangle of the bottom.

The well depth Wh may be larger than the depth of field of a detector, which will be described later, used for the microfluidic device 1, and 100 times or less the depth of field of the detector. Preferably, the well depth Wh may be 3 time or more and 10 times or less the depth of field of the detector.

The well 3 preferably has a volume that is 100 fL (femtoliter) or more. The upper limit of the volume of the well 3 may, for example, be 5,000 fL. The well 3 preferably has a volume of 4,000 fL or less, more preferably 3,000 fL or less, even more preferably 2,500 fL or less, and most preferably 2,000 fL.

Although not limited to, the microwell array 4 may be configured to transmit light. A hydrophilic or hydrophobic material may be selected as a material for forming the microwell array 4, considering properties desired to be imparted to the well 3 (to the inner surface of the well 3 in particular). The material for forming the microwell array 4 may, for example, be at least one of glass, resin and metal, or a mixture of these materials.

The configuration of the microwell array 4 of the present embodiment will be described in detail. As shown in Figs. 2 and 3, the microwell array 4 includes a plate-like bottom layer 11 and a plate-like wall layer 12 which is overlaid on the bottom layer 11.

In the bottom layer 11, the surface on which the wall layer 12 is disposed configures the bottom 9 of each well 3. More specifically, in the surface of the bottom layer 11 on which the wall layer 12 is disposed, regions where the wall layer 12 is not disposed configure the bottoms 9 of the respective wells 3. For example, the bottom layer 11 may be made of glass or a resin. The wall layer 12 has a plurality of holes passing through the wall layer 12 in the thickness direction and arranged in an array pattern as viewed in the thickness direction. Each hole has an inner surface configuring an inner peripheral surface 10 of a well 3. For example, the wall layer 12 may be made of a resin.

As shown in Figs. 2 and 3, the bottom layer 11 and the wall layer 12 may be separately formed, or may be integrally formed, for example, by injection-molding a resin. The wells 3 of the microwell array 4 may be formed, for example, by photolithography, or may be formed simultaneously with the injection molding mentioned above.

As shown in Figs. 1 and 2, the microwell array 4 is provided with a cover member 5 that configures a channel 2 of the microfluidic device 1 together with the microwell array 4. The cover member 5 is formed into a plate-like or sheet-like shape and disposed facing the aperture surface 6 of the microwell array 4. A gap serving as the channel 2 is defined between the microwell array 4 and the cover member 5.

The cover member 5 includes a plurality of through holes 15 passing therethrough in the thickness direction. The through holes 15 communicate with the channel 2 of the microfluidic device 1 to each serve as an inlet for charging fluid into the channel 2 or as an outlet for discharging fluid therefrom. In the present embodiment, the cover member 5 has two through holes 15A and 15B (first through hole 15A and second through hole 15B). The two through holes 15A and 15B are respectively disposed at two diagonally opposite corners of the rectangular well-forming region 8.

Each through hole 15 includes a small hole 16 and a large hole 17 which is larger than the small hole 16 as viewed in a direction in which the through hole is formed. The small hole 16 and the large hole 17 are aligned in a direction in which the through hole 15 is formed. The small hole 16 is open on the channel 2 side of the microfluidic device 1 to configure an opening end of the through hole 15. The large hole 17 is open on a side facing away from the channel 2 of the microfluidic device 1 to configure another opening end of the through hole 15. The large hole 17 serves, for example, as a fluid pool for pooling fluid that has flowed out of the channel 2 of the microfluidic device 1. The plurality of through holes 15 may, for example, be formed with the same shape and size, or may be formed with different shapes and sizes.

Although not limited thereto, the cover member 5 may, for example, be configured to transmit light. For example, in the case where an optical method is used for detecting fluorescence signals or the like caused by the presence of objects to be detected, such as particles or biomarkers, which are held in the wells 3, at least one of the cover member 5 and the microwell array 4 may be configured to transmit light. For example, in the case where a method other than an optical method is used for detecting fluorescence signals or the like caused by the presence of objects to be detected, such as particles or biomarkers, which are held in the wells 3, the cover member 5 and the microwell array 4 do not have to be configured to transmit light.

The material for forming the cover member 5 may, for example, be at least one of glass, resin and metal, or a mixture of these materials. The cover member 5 may have a rigidity for not being deformed by the flow of fluid or the like in

the channel 2.

Between the microwell array 4 and the cover member 5, a spacer 18 is provided, surrounding the well-forming region 8 of the microwell array 4. The spacer 18 defines the gap between the microwell array 4 and the cover member 5 and configures the channel 2 of the microfluidic device 1 together with the microwell array 4 and the cover member 5.

The spacer 18 may, for example, be formed integrally with the microwell array 4 or the cover member 5. In the present embodiment, however, the spacer 18 is formed separately from them. Examples of the material for the spacer 18 include, but are not particularly limited to, a double-sided adhesive tape formed by laminating a tackifier (e.g., acrylic tackifier) on both surfaces of a core film made of silicone rubber or an acrylic foam, and paper in which an adhesive is applied to both surfaces.

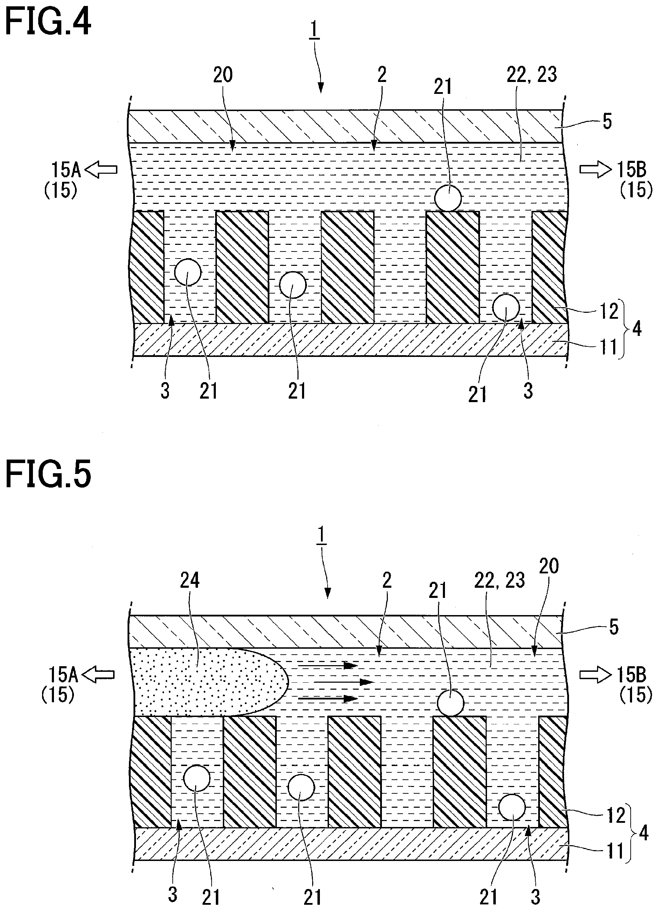

A usage example of the microfluidic device 1 mentioned above will be described. Figs. 4 to 6 are diagrams illustrating a usage example of the microfluidic device 1.

First of all, a particle-containing fluid 20 and a sealing liquid 24 are prepared. The particle-containing fluid 20 contains particles 21 as target substances. The particle-containing fluid 20 comprises the particles 21, a solvent fluid 22, and a detection reagent 23. The detection reagent 23 causes a biochemical reaction of target substances and is appropriately selected depending on the target substances. The sealing liquid 24, for example, is an oil. An analysis kit of the present embodiment includes the microfluidic device 1, the detection reagent 23, and the sealing liquid 24 mentioned above.

The particles 21 as target substances, which are objects to be analyzed, may, for example, be DNA, RNA, proteins, viral particles, bacteria, cells, or exosomes. The particles 21 may be trapped materials, such as beads, in which objects to be analyzed, such as DNA, RNA, proteins, viral particles, bacteria, cells, or exosomes, mentioned above are trapped. When viral particles, bacteria, cells, or exosomes are concerned, the reagent reacting in each well 3 can detect not only a protein or a sugar chain of the outer skin, but also DNA, RNA or a protein contained inside. Beads trapping objects to be analyzed may each have a surface that contains a substance identifying a protein or a sugar chain of the outer skin of a viral particle, a bacterium, a cell, an exosome, or the like.

Subsequently, the microfluidic device 1 is placed such that the flow of the particle-containing fluid 20 and the sealing liquid 24 in the channel 2 is directed in the horizontal direction and that the apertures of the wells 3 are oriented upward in the vertical direction. Then, the particle-containing fluid 20 is injected into the channel 2 from the first through hole 15A of the microfluidic device 1 by using a pipette. Fig. 4 shows a state where injection of the particle-containing fluid 20 has been completed.

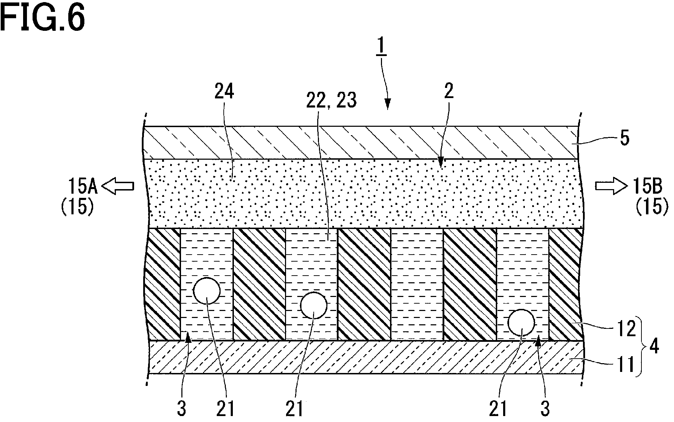

After that, as shown in Figs. 5 and 6, the sealing liquid 24 is injected into the channel 2 from the first through hole 15A of the microfluidic device 1 to fill the channel 2 with the sealing liquid 24. In other words, the particle-containing fluid 20 in the channel 2 is replaced by the sealing liquid 24. Thus, the particle-containing fluid 20 in the wells 3 is sealed by the sealing liquid 24. After sealing, for example, the microfluidic device 1 is heated to cause a biochemical reaction inside.

The biochemical reaction may be an enzymatic reaction. The enzymatic reaction may be an isothermal reaction or may be an anisothermal reaction, such as PCR, that requires heat cycles.. However, from the perspective of simplifying the device structure or the analysis procedure, the enzymatic reaction may preferably be an isothermal reaction. Comparing with anisothermal reaction, isothermal reaction can achieve a stable enzymatic reaction and is likely to have high reproducibility.

Specific examples of assays based on isothermal reaction may include, but are not limited to, invasive cleavage assay (ICA), loop-mediated isothermal amplification (LAMP) and SmartAmp.

After reaction, signals, as objects to be measured, are generated in the wells 3 of the microwell array 4. These signals may be fluorescence, light emission, phosphorescence, and the like. These signals have wavelength ranges observable as electromagnetic waves, such as visible light, ultraviolet light or infrared light. Accordingly, a detector including a device that can measure such wavelength ranges can be used as a detector of the present embodiment. Specifically, in the present embodiment, a fluorescence microscope 40 is used as a detector for detecting a reaction.

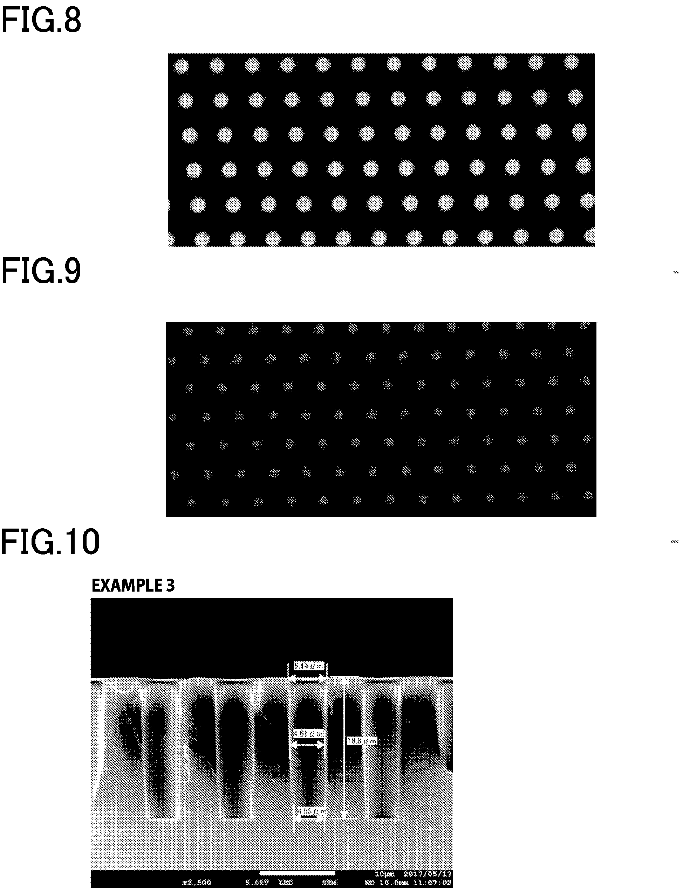

If the microwell array 4 is configured to transmit light, the microfluidic device 1 is disposed so as to be observable from the microwell array 4 side by the fluorescence microscope 40. Fig. 8 shows an example of an image observed by the fluorescence microscope 40. In the example shown in Fig. 8, fluorescence is observed by the fluorescence microscope 40.

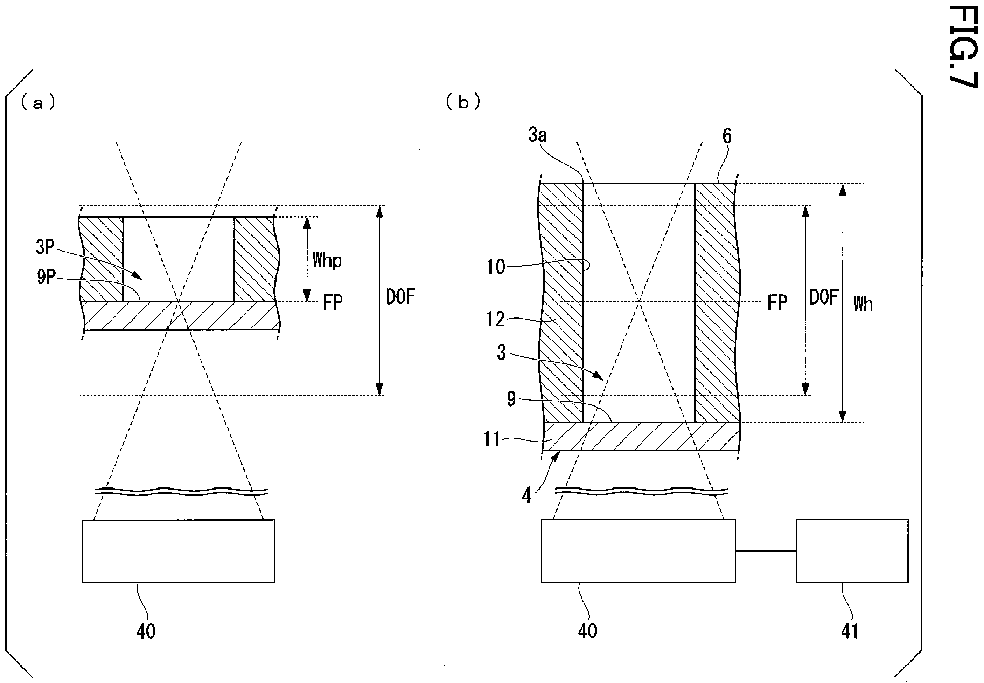

A relationship between the depth Wh of the wells 3 and a depth of field DOF of the fluorescence microscope 40 will be described in more detail. The term depth of field refers to a range in which the focus is sharp when observing or imaging a sample surface using the microscope, even when the distance between the objective lens and the sample surface is changed from the point of focus. In the present specification, the length of this range is taken to be a depth of field. Fig. 7 is a set of schematic diagrams each illustrating a well 3 and a depth of field DOF of a fluorescence microscope 40. Fig. 7(a) shows a well 3P based on conventional art and Fig. 7(b) shows a well 3 of the present embodiment.

As shown in Fig. 7(a), the conventional well 3P has a depth Whp which is set to be smaller than the depth of field DOF of the fluorescence microscope 40. The range that can be observed by the fluorescence microscope 40 is basically the range of the depth of field DOF. Thus, when the liquid in the well 3P is entirely fluorescent due to a biochemical reaction and a focal point FP of the fluorescence microscope 40 is adjusted to a bottom 9P of the well 3P, the fluorescence

microscope 40 observes fluorescence of the liquid in the well 3P, within the range of the well depth Whp that is smaller than the depth of field DOF. In other words, the fluorescence microscope 40 observes a state in which fluorescence signals are accumulated, within the range of the well depth Whp.

In this regard, as shown in Fig. 7(b), the well 3 of the present embodiment has a depth Wh which is set to be larger than the depth of field DOF of the fluorescence microscope 40 as a detector. Thus, when the liquid in the well 3 is entirely fluorescent due to a biochemical reaction and a focal point FP of the fluorescence microscope 40 is adjusted to half the length of the well depth Wh, the fluorescence microscope 40 observes fluorescence of the liquid in the well 3P, throughout the range of the depth of field DOF. In other words, the fluorescence microscope 40 observes a state in which fluorescence signals are accumulated, throughout the range of the depth of field DOF. Thus, compared to the conventional wells 3P, the wells 3 of the present embodiment can each be observed by the fluorescence microscope 40, in a state where more fluorescence signals are accumulated therein. Thus, even low-concentration target substances, which could not be detected by devices of conventional art, can be detected by accumulating more fluorescence signals.

The example set forth above uses the particle-containing fluid 20 that contains the particles 21 as target substances. However, the fluid is not limited to this. As long as the fluid contains target substances, any fluid may be used, i.e. the fluid does not have to contain the particles 21.

The detector may include an analysis unit 41 that analyzes a measured fluorescence value or other values (measured values). As the analysis unit 41, for example, a plate reader, a spectrophotometer, or the like may be used. The analysis system of the present embodiment includes the microfluidic device 1, the fluorescence microscope (detector) 40, and the analysis unit 41 described above.

According to the present embodiment, the microfluidic device 1 is an analysis device that detects a biochemical reaction by using the fluorescence microscope 40. The microfluidic device 1 is provided with the microwell array 4 in which a plurality of wells 3 are formed. Each well 3 may have a volume of 100 fL or more. The depth Wh of each well 3 may be larger than the depth of field DOF of the fluorescence microscope 40 and 100 times or less the depth of field DOF thereof.

In the microfluidic device 1 described above, if the depth Wh of the well 3 is larger than the depth of field DOF of the fluorescence microscope 40, more signals such as of fluorescence generated by biochemical reaction of target substances, if any, can be accumulated in a direction of the depth Wh compared to the conventional configuration. Thus, target substances having low concentration can be reliably detected.

In the following, the present invention will be described in detail by way of examples; however the present invention should not be limited by these examples.

[Examples]

[Example 1]

In the present example, the effects of accumulating fluorescence signals were researched by changing the depth of the wells.

(Conditions)

The wells of the microfluidic device were all formed into a cylindrical shape. Each well had a diameter (aperture diameter) of 5 μm and a depth of 20 μm (designed values). A microfluidic device having the wells of the above configuration was prepared from a thermoplastic resin by injection molding.

For a reagent 1, a liquid mixture of fluorescein, MOPS (3-morpholinopropanesulfonic acid), $MgCl_2$, Tween, and DDW (purified water) was prepared. More specifically, the liquid mixture prepared as a reagent 1 contained 20 μM fluorescein, 10 mM MOPS, 10 mM $MgCl_2$, 0.05% Tween 20, and DDW. As a sealing liquid 24, an oil (FC40 manufactured by Sigma-Aldrich) was used.

(Procedure)

First, the reagent 1 (20 μL) prepared as described above was supplied to the first through hole of the microfluidic device by using a pipette. After that, the oil (150 μL) was supplied to the first through hole of the microfluidic device for sealing. Then, the device was observed by a fluorescence microscope (manufactured by Keyence Corporation). The fluorescence microscope had a depth of field of 3 μm (±1.5 μm).

(Results)

Fig. 8 shows an image of a fluorescence field captured by the fluorescence microscope of Example 1. In Example 1, the fluorescence value (relative value) was about 3,500.

[Comparative Example 1]

The microfluidic device used in the present comparative example had wells each having a depth 3 μm, which was the only change in conditions compared to those of Example 1. The procedure is the same as in Example 1.

Fig. 9 shows an image of a fluorescence field captured by the fluorescence microscope of Comparative Example 1. In Comparative Example 1, the fluorescence value (relative value) was about 1,100.

As can be seen from the results set forth above, the microfluidic device of Example 1 could accumulate 3 times or more the fluorescence values compared to Comparative Example 1.

[Example 2]

In the present example, detectable low-concentration target substances were researched by changing the depth of the wells.

(Conditions)

The wells of the microfluidic device were all formed into a cylindrical shape. Each well had a diameter (aperture diameter) of 5 μm and a depth of 20 μm (designed values). A microfluidic device having the wells of the above configuration was prepared from a thermoplastic resin by injection molding.

For a reagent 2, a liquid mixture of two types of allele probes, one type of Invader oligo, two types of FRET cassettes, MOPS, $MgCl_2$, flap endonuclease, Tween, DDW, and two types of targets was prepared. More specifically, the liquid mixture prepared as a reagent 2 contained 1 μM of allele probe 1, 1 μM of allele probe 2, 1 μM of Invader oligo, target nucleic acid 1, target nucleic acid 2, which are shown in the following Table 1, and two types of FRET cassettes for detection (final concentration of 2 μM), 10 mM MOPS, 10 mM $MgCl_2$, 1,000U/μL flap endonuclease, 0.05% Tween 20, and DDW. Five types of reagent 2 were prepared so that the target nucleic acids 1 and 2 therein would each have concentrations 0 fM, 0.2 fM (200 aM), 2 fM, 20 fM and 200 fM. As a sealing liquid 24, an oil (FC40 manufactured by Sigma-Aldrich) was used.

[Table 1]

| Name | Seq. No. | Base sequence (5'→3') |
|---|---|---|
| Target nucleic acid 1 | 1 | CCGAAGGGCATGAGCTGCgTGATGAGCTGCACGGTGGAG GTGAGGCAGATGCCCAG |
| Target nucleic acid 2 | 2 | CCGAAGGGCATGAGCTGCaTGATGAGCTGCACGGTGGAG GTGAGGCAGATGCCCAG |
| Allele probe 1 | 3 | cgcgccgaggCGCAGCTCATGCCC |
| Allele probe 2 | 4 | acggacgcggagTGCAGCTCATGCCC |
| Invader oligo | 5 | CCACCGTGCARCTCATCAA |

In Table 1, the only difference between the target nucleic acid 1 (Seq. No. 1) and the target nucleic acid 2 (Seq. No. 2) as objects to be detected lies in the decapitalized g (guanine) and a (adenine) of the base sequences.

The allele probe 1 (Seq. No. 3) has a sequence that is complementary to the target nucleic acid 1 and forms a duplex. However, the decapitalized part 5'-cgcgccgagg-3' (Seq. No. 6) does not form a base pair, but forms a flap and is cut out as a nucleic acid fragment by the flap endonuclease.

The allele probe 2 (Seq. No. 4) has a sequence that is complementary to the target nucleic acid 2 and forms a duplex. However, the decapitalized part 5'-acggacgcggag-3' (Seq. No. 7) does not form a base pair, but forms a flap and is cut out as a nucleic acid fragment by the flap endonuclease.

Subsequently, each cut-out nucleic acid fragment is hybridized with a FRET cassette to form a flap and become a substrate of the flap endonuclease, and is cut off. As a result, a fluorescence signal is detected.

(Procedure)

First, the reagent 2 (20 μL) prepared as described above was supplied to the first through hole of the microfluidic device by using a pipette. After that, the oil (150 μL) was supplied to the first through hole of the microfluidic device for sealing. Then, the microfluidic device was heated for isothermal reaction to generate fluorescence signals. Then, the wells

were observed by a fluorescence microscope (manufactured by Keyence Corporation). The fluorescence microscope had a depth of field of 3 μm (±1.5 μm).

In Example 2, the wells formed in the microfluidic device were arbitrarily brought into a field of view of the fluorescence microscope and five images were captured for the respective target concentrations. Finally, in the captured five images, the wells that had generated fluorescence signals (which may be termed fluorescence wells hereinafter) were counted to calculate an average value.

(Results)

The results are shown in Table 2. As shown in Table 2, invader reactions were confirmed in the reagents 2 respectively having target concentrations of 0 fM, 0.2 fM, 2 fM, 20 fM and 200 fM. As will be understood, as the target concentration decreases from 200 fM to 0.2 fM at a ratio of 1/10, the average value of the counts of the fluorescence wells also decreases at a ratio of about 1/10. It should be noted that, the average value of the counts of the fluorescence wells being 1 or less means that fluorescence wells cannot be confirmed. Accordingly, it was confirmed that targets were detectable for the concentration as low as 2 fM.

[Table 2]

| Target concentration (fM) | Count of fluorescence wells (average) |
|---|---|
| 0.0 | 0.0 |
| 0.2 | 0.4 |
| 2.0 | 3.4 |
| 20.0 | 34.0 |
| 200.0 | 326.8 |

(Comparative Example 2)

The microfluidic device used in the present comparative example had wells each having a depth 3 μm, which was the only change in conditions compared to those of Example 2. Six types of reagent 2 were prepared so that the target nucleic acids 1 and 2 therein would each have concentrations 0 fM, 20 fM, 200 fM, 2,000 fM (2 pM), 20,000 fM (20 pM) and 200,000 fM (200 pM).

(Procedure)

First, the reagent 2 (20 μL) prepared as described above was supplied to the first through hole of the microfluidic device by using a pipette. After that, oil (150 μL) was supplied to the first through hole of the microfluidic device for sealing. Then, the microfluidic device was heated to perform the fluorescence reaction. Then, the wells were observed by a fluorescence microscope (manufactured by Keyence Corporation). The fluorescence microscope had a depth of field of 3 μm (±1.5 μm).

As in Example 2, the wells formed in the microfluidic device were arbitrarily brought into a field of view of the fluorescence microscope and five images were captured for the respective target concentrations. Finally, in the captured five images, fluorescence wells were counted to calculate an average value.

(Results)

The results are shown in Table 3. As shown in Table 3, invader reactions were confirmed in the reagents 2 in which the target nucleic acids 1 and 2 each had the concentrations of 0 fM, 20 fM, 200 fM, 2,000 fM, 20,000 fM and 200,000 fM. As will be understood from Table 3, as the target concentration decreases from 2,000 fM to 20 fM at a ratio of 1/10, the average value of the counts of the fluorescence wells also decreases at a ratio of about 1/10. However, since the average value of the counts of the fluorescence wells at 20 fM is 7.4, the average value of the counts of the fluorescence wells is assumed to be 1 or less when the target concentration is reduced to 1/10 thereof, i.e. 2 fM, which means that no fluorescence wells can be confirmed. Accordingly, it is considered that, in Comparative Example 2, target substances cannot be appropriately detected at a concentration of 2 fM or less.

[Table 3]

| Target concentration (fM) | Count of fluorescence wells (average) |
|---|---|
| 0 | 0.4 |
| 20 | 7.4 |
| 200 | 61.8 |
| 2,000 | 606.2 |
| 20,000 | 4,997.0 |
| 200,000 | 9,994.2 |

As will be understood from the results set forth above, the microfluidic device of Example 2 could achieve detection sensitivity that is about 10 times that of the microfluidic device of Comparative Example 2.

As a result of researches conducted by the inventors, the following findings were obtained in addition to those of the examples described above.

When fluorescence wells in a predetermined range are counted after increasing volumes of the wells, low-concentration target substances may be detected. In this case, if target substances at a concentration, for example, of 10 fM are to be detected, count per field (e.g., region including 100 $\times$ 100 wells if the wells are arrayed in a square lattice pattern) will be about 6. Therefore, it is preferable that each well has a volume of 100 fL or more. Furthermore, each well preferably has a large aspect ratio (ratio of the well depth to the aperture diameter of the well).

When each well has a larger depth, more light can be collected. Each well preferably has a depth that is larger than the depth of field of the detector, such as a microscope. When each well has a large depth, light from the fluorescent substances outside the focal length can be accumulated.

When one molecule of DNA is an object to be detected and if the number F of fluorescent molecules produced per unit time due to enzymatic reaction is constant, each well having a volume V (the volume herein is based on a cylindrical well with a diameter a and a depth h) will hold the fluorescent molecules therein at a concentration Da which is expressed by the following Formula (2).

$$Da=F/(\pi\times a^2\times h/4) \qquad \ldots (2)$$

The fluorescence signals, which can be accumulated by a fluorescence microscope, are fluorescent molecules that are present in the observation direction (depth direction of wells) within a range that is shorter than 100 times of the depth of field, while fluorescent brightness (fluorescence intensity) to be detected corresponds to the number of fluorescent molecules per unit volume on a plane that is perpendicular to the observation direction. A fluorescence brightness Q in this case can be expressed by the following Formula (3).

$$Q=F/(\pi\times a^2/4) \qquad \ldots (3)$$

Accordingly, if the volume V of a well is unchanged, it is preferable that the diameter a is decreased and the depth h is increased to accumulate fluorescence signals (to increase the amount of fluorescence to be measured). The ratio between the diameter a and the depth h may preferably be $h/a\geq1$, and more preferably $h/a\geq1.5$.

The volume of the wells per unit volume, which can be imaged by one exposure of the microscope, may be increased to increase the probability for the target molecules to enter the wells. With this configuration, low-concentration samples can be detected. To increase the volume of the wells, it is necessary not only to arrange the wells so as to be close to each other, but also to increase the volume of each well. For closely arranging the wells, each well may preferably have a smaller diameter, with the depth being increased accordingly for increase of the volume.

If the volume of the wells per surface area is increased, reactivity of the enzymatic reaction may improve. For example, if the diameter of each well is changed, or if the aspect ratio of each well is changed, reactivity may improve. The enzymatic reaction occurring in a fine space such as of a well will receive a non-negligible influence from the surface of the solid layer. Therefore, the surface area of the inner wall of each well per volume of the well may be reduced to reduce the influence from the surface of the inner wall of the well due to adsorption or the like of enzymes or targets thereto. To reduce the surface area ratio of the reaction volume (volume of each well), the volume of each well is required to be increased, which means that the diameter of each well is required to be increased. Therefore, this increase of diameter is required to be adjusted with the accumulation of light by increasing the depth of each well as described above. Thus, if the reaction volume is 5,000 fL or more, each well may preferably have a surface area ratio (surface area of well / volume of well) of 0.4 or less. If the reaction volume is 5,000 fL or less, each well may preferably have a surface area ratio of 1 or less.

The oil used as a sealing liquid has a high probability of inhibiting the enzymatic reaction. Thus, if the volume of each well is unchanged, the diameter thereof may preferably be reduced to reduce the area contacting the oil. Alternatively, the depth of each well may be increased to increase the distance between the bottom and the oil to thereby prevent the reaction from being inhibited. The distance between the bottom and the oil is preferably 20 μm or more, and more preferably 100 μm or more. If the distance between the bottom and the oil is 3 μm or less, the probability of inhibiting the reaction is high.

If the biochemical reaction is a heating reaction, it is necessary to consider evaporation of the very small quantity of reaction liquid held in the wells. If the wells each have a reaction volume of 100 fL or less, biochemical reaction may be considered to have ceased due to evaporation of the reaction liquid from the wells.

[Examples 3 to 5 and Comparative Examples 3 and 4]

Analysis devices (microfluidic devices) including well arrays of Examples 3 to 5 and Comparative Examples 3 and 4 were prepared. These devices had wells with different depths and different aperture diameters. Each analysis device had the same shape as the device shown in Figs. 1 and 2. The wells were each formed into a cylindrical shape.

Subsequently, using the nucleic acid fragments shown in Table 1, nucleic acids were detected based on an invasive cleavage assay under the conditions set forth below.

Specifically, as a reagent 3, a liquid mixture was prepared, which contained 0.1 μM allele probe 1, 0.1 μM allele probe 2, 1 μM Invader oligo, and 10 fM target nucleic acid 1 shown in Table 1, and two types of FRET cassettes for detection (final concentration of 2 μM), 10 mM MOPS, 20 mM $MgCl_2$, 0.03 mg/mL flap endonuclease, 0.05% Tween 20, and DDW. As a sealing liquid 24, an oil (FC40 manufactured by Sigma-Aldrich) was used.

Although the target nucleic acid 2 was not used for the reagent 3, the allele probe 2 and the FRET cassettes conforming to the allele probe 2 were also mixed to create experimental conditions approximate to those for actually detecting a base mutation.

Then, the reagent 3 (20 μL) prepared as described above was supplied to the first through hole of each microfluidic device by using a pipette. Then, 150 μL of oil (FC40 manufactured by Sigma-Aldrich) was supplied to the first through hole of each microfluidic device for sealing. Then, each microfluidic device was heated to produce an isothermal reaction to generate fluorescence signals. Furthermore, the well array of each microfluidic device was observed by a fluorescence microscope (manufactured by Keyence Corporation) to measure the fluorescence intensity (relative value) of the signals.

More specifically, the wells formed in each microfluidic device were observed by a fluorescence microscope to capture an image by arbitrarily bringing the wells into a field of view. In the captured image, fluorescence intensities (relative values) of the wells that had generated significant signals were measured to calculate an average value thereof as a signal value. At the same time, fluorescence intensities (relative values) of the wells that had not generated significant signals were measured to calculate an average value thereof as a noise value. The fluorescence microscope had a depth of field of 3 μm (±1.5 μm).

The series of experiments mentioned above were conducted for the analysis devices having well arrays of Examples 3 to 5 and Comparative Examples 3 and 4 to measure fluorescence intensities (relative values) of signals and noises.

The analysis devices having well arrays of Examples 3 to 5 and Comparative Examples 3 and 4 were each cut along a plane, which was parallel to the depth direction of the wells and included centers of the wells, for observation by a scanning electron microscope to actually measure the aperture diameters and depths of the wells. The aperture diameter of each well was the diameter at the aperture of the well.

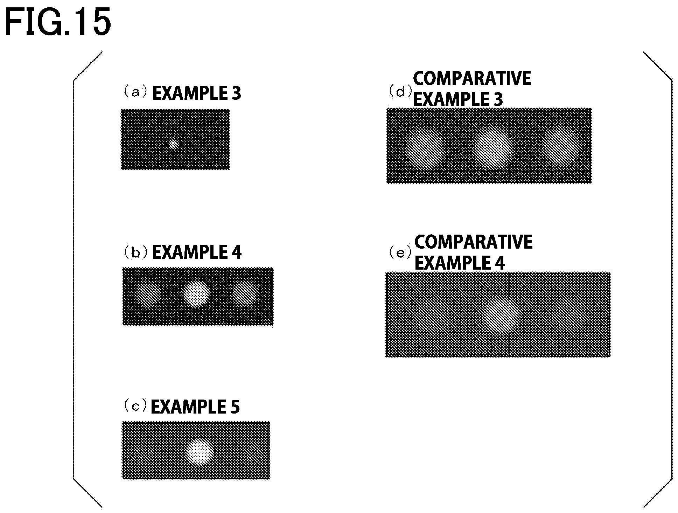

Figs. 10 to 14 respectively show scanning electron micrographs of the well arrays of the analysis devices of Examples 3 to 5 and Comparative Examples 3 and 4. Fig. 15 is a set of images in which (a) to (e) show typical fluorescence signals detected by using the analysis devices of Examples 3 to 5 and Comparative Examples 3 and 4.

Table 4 below shows measurements of the well arrays of the analysis devices of Examples 3 to 5 and Comparative Examples 3 and 4, in terms of aperture diameter, well depth, aspect ratio, well volume, average signal (relative values), average noise (relative value) and signal/noise ratio (S/N ratio).

[Table 4]

| | Example 3 | Example 4 | Example 5 | Comparative Example 3 | Comparative Example 4 |
|---|---|---|---|---|---|
| Aperture diameter (μm) | 5.14 | 10.20 | 10.70 | 16.00 | 16.20 |
| Depth (μm) | 18.80 | 14.30 | 18.90 | 13.50 | 14.70 |
| Aspect ratio | 3.66 | 1.40 | 1.77 | 0.84 | 0.91 |

(continued)

| | Example 3 | Example 4 | Example 5 | Comparative Example 3 | Comparative Example 4 |
|---|---|---|---|---|---|
| Volume (fL) | 390.10 | 1,168.49 | 1,699.49 | 2,714.34 | 3,029.96 |
| Signal (relative value) | 1,700 | 1,040 | 1,100 | 879 | 950 |
| Noise (relative value) | 670 | 484 | 410 | 564 | 520 |
| S/N ratio | 2.54 | 2.15 | 2.68 | 1.56 | 1.83 |

From the results, it was obvious that the analysis devices of Examples 3 to 5 having an aspect ratio of 1 or more showed high signal/noise ratio and could favorably detect low-concentration target substances.

Some preferred embodiments of the present invention have been described so far, but the present invention should not be limited to the embodiments described above. Addition, omission, substitution, and other changes of a configuration can be made, limited only by the scope of the appended claims.

[Reference Signs List]

| | |
|---|---|
| 1 | Microfluidic device (analysis device) |
| 3 | Well |
| 4 | Microwell array |
| 23 | Detection reagent |
| 24 | Sealing liquid |
| 40 | Fluorescence microscope (detector) |
| 41 | Analysis unit |
| DOF | Depth of field |
| FP | Focal point |
| Wd | Aperture diameter |
| Wh | Well depth |

## Claims

**1.** An analysis device (1) for detecting biochemical reactions using a fluorescence microscope (40) wherein

the device includes a well array (4) in which a plurality of wells (3) are formed; and each of the wells has a volume of 100 fL to 5,000 fL,
each of the wells (3) has a surface area ratio (surface area of well / volume of well) of 1 or less, ; and
each of the wells (3) has an aspect ratio of 1 or more as calculated by the following Formula (1)
Aspect ratio = Depth of each well / Diameter of largest circle among circles included in a region enclosed by a rim of each well ... Formula (1);
wherein the well array (4) is provided with a cover member (5) that configures a channel (2) of the analysis device (1) together with the well array (4) and
**characterized in that**
the cover member (5) includes a plurality of through holes (15) passing therethrough in the thickness direction, wherein the through holes (15) communicate with the channel (2) to serve as a fluid inlet or as a fluid outlet therefrom, each through hole (15) including a small hole (16) and a large hole (17) which is larger than the small hole (16) as viewed in a direction in which the hole (15) is formed, and wherein the small hole (16) is open on the channel (2) side of the microfluidic device (1), and the large hole (17) is open on a side facing away from the channel (2) of the microfluidic device (1) and suitable for serving as a fluid pool.

**2.** The analysis device according to claim 1, wherein each of the wells has a depth that is larger than a depth of field of the fluorescence microscope and 100 times or less the depth of field.

**3.** The analysis device according to claim 2, wherein the depth of each of the wells is 3 times or more the depth of field and 10 times or less the depth of field.

4. An analysis kit comprising the analysis device according to any one of claims 1 to 3, a detector, a detection reagent (23), and a sealing liquid (24).

5. An analysis system comprising the analysis kit of claim 4, the fluorescence microscope, and an analysis unit (41) that analyzes values measured by the fluorescence microscope.

## Patentansprüche

1. Analysevorrichtung (1) zum Nachweisen von biochemischen Reaktionen unter Verwendung eines Fluoreszenzmikroskops (40), wobei

   die Vorrichtung ein Well-Array (4) einschließt, in dem eine Vielzahl von Vertiefungen (3) ausgebildet ist; und jede der Vertiefungen ein Volumen von 100 **fL** bis 5.000 **fL** aufweist,
   jede der Vertiefungen (3) ein Oberflächenverhältnis (Oberflächenbereich von Vertiefung / Volumen von Vertiefung) von 1 oder weniger aufweist; und
   jede der Vertiefungen (3) ein Seitenverhältnis von 1 oder mehr aufweist, das durch die folgende Formel (1) berechnet wird
   Seitenverhältnis = Tiefe jeder Vertiefung / Durchmesser des größten Kreises unter den Kreisen, die in einem von einem Rand jeder Vertiefung eingeschlossenen Bereich eingeschlossen sind ... Formel (1);
   wobei das Well-Array (4) mit einem Abdeckelement (5) bereitgestellt ist, das einen Kanal (2) der Analysevorrichtung (1) zusammen mit dem Well-Array (4) konfiguriert und **dadurch gekennzeichnet ist, dass** das Abdeckelement (5) eine Vielzahl von Durchgangslöchern(15) einschließt, die in der Dickenrichtung hindurchgehen, wobei die Durchgangslöcher (15) mit dem Kanal (2) kommunizieren, um als ein Fluideinlass oder Fluidauslass daraus zu fungieren, wobei jedes Durchgangsloch (15) ein kleines Loch (16) und ein großes Loch (17), das größer ist als das kleine Loch (16), einschließt, betrachtet in einer Richtung, in der das Loch (15) ausgebildet ist, wobei das kleine Loch (16) auf der Seite von Kanal (2) der Mikrofluidvorrichtung (1) offen ist, und das große Loch (17) auf einer dem Kanal (2) abgewandten Seite der Mikrofluidvorrichtung (1) offen ist und geeignet ist, als ein Fluidreservoir zu fungieren.

2. Analysevorrichtung nach Anspruch 1, wobei jede der Vertiefungen eine Tiefe aufweist, die größer ist als eine Schärfentiefe des Fluoreszenzmikroskops und das 100-fache oder weniger der Schärfentiefe beträgt.

3. Analysevorrichtung nach Anspruch 2, wobei die Tiefe jeder der Vertiefungen das 3-fache oder mehr der Schärfentiefe und das 10-fache oder weniger der Schärfentiefe beträgt.

4. Analysekit umfassend die Analysevorrichtung nach einem beliebigen der Ansprüche 1 bis 3, einen Detektor, ein Nachweisreagens (23) und eine Dichtungsflüssigkeit (24).

5. Analysesystem umfassend den Analysekit nach Anspruch 4, das Fluoreszenzmikroskop und eine Analyseeinheit (41), die von dem Fluoreszenzmikroskop gemessene Werte analysiert.

## Revendications

1. Un dispositif d'analyse (1) pour détecter des réactions biochimiques à l'aide d'un microscope à fluorescence (40) dans lequel

   le dispositif comporte un réseau de puits (4) dans lequel une pluralité de puits (3) sont formés ; et chacun des puits a un volume de 100 fL à 5 000 fL,
   chacun des puits (3) a un rapport de surface (surface du puits / volume du puits) de 1 ou moins ; et
   chacun des puits (3) a un rapport d'aspect de 1 ou plus tel que calculé par la formule (1) suivante
   Rapport d'aspect = profondeur de chaque puits / diamètre du plus grand cercle parmi des cercles inclus dans une région délimitée par un rebord de chaque puits... Formule(1) ;
   dans lequel le réseau de puits (4) est pourvu d'un élément de recouvrement (5) qui configure un canal (2) du dispositif d'analyse (1) conjointement avec le réseau de puits (4) et
   **caractérisé en ce que**
   l'élément de recouvrement (5) comporte une pluralité de trous traversants (15) traversant celui-ci dans la

direction d'épaisseur, dans lequel les trous traversants (15) communiquent avec le canal (2) pour servir d'entrée de fluide ou de sortie de fluide à partir de celui-ci, chaque trou traversant (15) comportant un petit trou (16) et un grand trou (17) qui est plus grand que le petit trou (16) vu dans une direction dans laquelle le trou (15) est formé, et dans lequel le petit trou (16) est ouvert du côté du canal (2) du dispositif microfluidique (1), et le grand trou (17) est ouvert du côté opposé au canal (2) du dispositif microfluidique (1) et adapté pour servir de réservoir de fluide.

**2.** Le dispositif d'analyse selon la revendication 1, dans lequel chacun des puits a une profondeur qui est supérieure à une profondeur de champ du microscope à fluorescence et 100 fois supérieure ou moins à la profondeur de champ.

**3.** Le dispositif d' analyse selon la revendication 2, dans lequel la profondeur de chacun des puits est 3 fois supérieure ou plus à la profondeur de champ et 10 fois supérieure ou moins à la profondeur de champ.

**4.** Un kit d'analyse comprenant le dispositif d'analyse selon l'une quelconque des revendications 1 à 3, un détecteur, un réactif de détection (23) et un liquide d'étanchéité (24).

**5.** Un système d'analyse comprenant le kit d'analyse selon la revendication 4, le microscope à fluorescence et une unité d'analyse (41) qui analyse des valeurs mesurées par le microscope à fluorescence.

FIG.1

1
6
2
15A(15)
15B(15)
II
II
5
4
8
7

FIG.2

1
3
6
2
3
5
18
12
11
4
16
17
17
16
15A(15)
15B(15)

# FIG.3

(a)
4
3
10
9
3
3a
3
6
3
30
30
30
IIIb
IIIb
3
Wd
Wd
(b)
Wd
Wd
3a
6
Wh
12
11
3
9
3
10
3
4

# FIG.4

1
20
2
22, 23
21
5
15A
(15)
15B
(15)
12
4
11
3
21
21
21
3

# FIG.5

1
24
2
21
22, 23
20
5
15A
(15)
15B
(15)
12
4
11
3
21
21
21
3

# FIG.6

1
24
22, 23
2
5
15A
(15)
15B
(15)
12
4
11
3
21
21
21
3

FIG.7

(a)
(b)
DOF
Wh
FP
Whp
6
3a
10
12
3
9
11
4
3P
9P
40
41

FIG.8

FIG.9

FIG.10

EXAMPLE 3

5.14 μm
4.61 μm
18.8 μm
4.05 μm
10μm 2017/05/17
x2,500 5.0kV LED SEM WD 10.0mm 11:07:02

FIG.11

EXAMPLE 4

10.2 μm
10.0 μm
14.3 μm
9.74 μm
10μm 2017/05/01
x2,300 5.0kV LED SEM WD 9.4mm 14:45:55

FIG.12

EXAMPLE 5

10.7 μm
10.0 μm
18.9 μm
9.42 μm
10μm 2017/05/17
x2,000 5.0kV LED SEM WD 10.0mm 10:56:07

# FIG.13

COMPARATIVE EXAMPLE 3

16.0 μm
15.5 μm
13.5 μm
15.2 μm
1μm 2017/03/28
x3,000 5.0kV LED SEM WD 10.3mm 15:15:23

# FIG.14

COMPARATIVE EXAMPLE 4

16.2 μm
14.7 μm
15.5 μm
10kV X3,000 5μm 0000 10 13 SEI

# FIG.15

(a) EXAMPLE 3

(b) EXAMPLE 4

(c) EXAMPLE 5

(d) COMPARATIVE EXAMPLE 3

(e) COMPARATIVE EXAMPLE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014503831 T
- JP 2017049151 A
- WO 2016196229 A1